(19) Europäisches Patentamt

European Patent Office

Office européen des brevets

(11) Publication number: **0 246 009 B1**

(12) **EUROPEAN PATENT SPECIFICATION**

(45) Date of publication of patent specification: **03.02.93**   (51) Int. Cl.⁵: **C09J 7/02**, //A61F13/15

(21) Application number: **87303892.1**

(22) Date of filing: **30.04.87**

The file contains technical information submitted
after the application was filed and not included in
this specification

(54) **Pressure-sensitive adhesive tape having improved toughness.**

(30) Priority: **07.05.86 US 860557**

(43) Date of publication of application:
**19.11.87 Bulletin 87/47**

(45) Publication of the grant of the patent:
**03.02.93 Bulletin 93/05**

(84) Designated Contracting States:
**DE ES FR GB IT**

(56) References cited:
**EP-A- 0 235 930**
**DE-A- 1 494 023**
**US-A- 4 137 362**
**US-A- 4 237 889**
**US-A- 4 451 533**

(73) Proprietor: **MINNESOTA MINING AND MANU-
FACTURING COMPANY**
**3M Center, P.O. Box 33427**
**St. Paul, Minnesota 55133-3427(US)**

(72) Inventor: **Sipinen, Alan J. c/o Minnesota Min-
ing and**
**Manufacturing Company 2501 Hudson Road**
**P.O. Box 33427 St. Paul Minnesota 55133(US)**

(74) Representative: **Baillie, Iain Cameron et al**
**c/o Ladas & Parry, Altheimer Eck 2**
**W-8000 München 2(DE)**

## EP 0 246 009 B1

**Description**

This invention relates to a pressure-sensitive adhesive tape comprising a backing of a substantially untensilized tough, ductile film. The tape is particularly useful for fasteners on disposable

Pressure-sensitive adhesive tapes used as closure tapes on disposable diapers require a special combination of properties. One widely used tape uses paper treated with moisture-resistant polymer as a backing. Paper is a surprisingly expensive material, and its cost is increased by the several treatments to which it is subjected in making the tape suitable for diaper closure use. Further, even treated paper retains a moisture-sensitivity which occasionally weakens it sufficiently to cause it to fail when used in a tape closure for a diaper worn by an active baby. Also, paper is comparatively stiff so that the tape does not flex with the diaper and the edge of a paper tape closure may injure a baby's tender skin.

Cloth-backed tapes can be more attractive, flexible, and tear-resistant than paper but cloth is expensive, special treatments are required to prevent the penetration of adhesive through it, and there is a tendency for it to ravel. Various types of nonwoven tape backings, including the so-called "spun-bonded" polymeric backings are less expensive to make and more ravel-resistant than woven cloth, but their thickness, tear-resistance, etc., are not uniform and their open nature makes application of adhesive coating difficult and expensive.

Film-backed pressure-sensitive adhesive tapes, using backings such as polypropylene, have proven to be generally quite effective for diaper closure tapes but greater latitude in the conditions under which they can be used has also been sought. For example, having tightly closed and adhered one side of a diaper, a person applying the diaper may need to pull strongly on the diaper tape projecting from the other side of the diaper to draw the diaper closed and adhere it in place. This pulling effect, if performed in a rapid or jerky manner, can rupture the tape.

The pressure-sensitive adhesive tapes of the invention have an improved balance of properties, including especially improved toughness needed for use as diaper closures.

EP-A-235,930 forms part of the state of the art against the present application by virtue of Article 54(3) EPC and discloses soft, pliable pressure-sensitive adhesive tapes having a backing of a substantially untensilized, tough, ductile film, the film being a blend of a crystalline isotactic polypropylene and a compatible ethylene-based polymer. The ethylene-based polymer may for example be ethylene-vinyl acetate, ethylene-acrylic acid or low or very low-density polyethylene. There is no disclosure of a backing containing a styrene-diene block copolymer.

US 4,137,362 discloses a pressure-sensitive adhesive tape having a tensilized backing composed of isotactic polypropylene and optionally a second component which may be polyethylene, an ethylene-propylene-diene terpolymer, polyisobutylene or an ethylene-vinyl acetate copolymer. The backing has a tensile strength of above 170 kg/cm$^2$ in the longitudinal direction and a tensile strength of above 800 kg/cm$^2$ in the transverse direction. The backing has a thickness of 10 to 300 um and the tape is easily cut with the fingers (implying a low longitudinal tear strength) and is intended to be used for sealing cartons.

The present invention provides a pressure-sensitive adhesive tape comprising a backing of a substantially untensilized, tough, ductile film, and a coating of a pressure-sensitive adhesive on one side of said film; said film comprising a blend of crystalline isotactic polypropylene and a compatible styrene-diene copolymer having a flexural modulus lower than that of said isotactic polypropylene, said film having

1) a longitudinal yield tensile strength of at least about 160 kg/cm$^2$;
2) a transverse yield tensile strength of at least about 140 kg/cm$^2$;
3) a longitudinal tear strength of at least about 50 g/ply;
4) a tensile impact toughness of greater than about 20 mm/sec.

Preferred features of the tape are defined in claims 2 to 9. The invention also provides a disposable diaper to which such tape is attached as a diaper closure means.

The amount of flexible styrene diene copolymer in the blend and the conditions under which the blend is extruded as a film, particularly the chill roll temperature and the length of time the film is in contact therewith, can be varied to achieve the desired toughness and tensile properties. In general, an increase in toughness and decrease in tensile strength is obtained by increasing the amount of flexible polymer, decreasing the chill roll temperature or increasing the chill roll contact time. Surprisingly, high tensile strengths such as listed above can be retained while also achieving high toughness.

The film preferably comprises a blend of a major amount by weight of crystalline, isotactic polypropylene and a minor amount of a compatible flexible styrene-diene polymer, the precise amount of flexible polymer depending on the chosen chill roll temperature and contact time.

The compatible copolymer may for example be a styrene-butadiene-styrene block copolymer or a styrene-isoprene-styrene block copolymer.

2

Styrene-diene block copolymers useful in this invention have a flexural modulus, as measured by ASTM-D-890, lower than that of the crystalline isotactic polypropylene. The lower flexural modulus will improve the toughness of the blend as compared with that of the crystalline isotactic polypropylene alone. Crystalline isotactic polypropylene generally has a flexural modulus of greater than about 500 megapascal (MPa = $10^6$ N/m$^2$) which means that suitable styrene-diene block copolymers will generally have a flexural modulus of less than about 500 MPa, preferably less than about 400 MPa. The flexural modulus is also preferably at least about 5 MPa, more preferably at least about 40 MPa.

In general, suitable flexible polymers are also thermoplastic, i.e., they soften or melt to a blendable viscosity. Many of the flexible polymers are elastomers, i.e. when stretched they will retract to assume their original dimensions or nearly so. In particular, preferred elastomers are defined by ASTM Special Technical Bulletin, No. 184, wherein a sample is stretched at room temperature to twice its original length, held for a five minute period and released whereupon the sample will return to within 10% of its original length within five minutes.

The styrene-diene block copolymer must be chemically and/or mechanically compatible with the crystalline isotactic polypropylene to permit effective blending during extrusion and to minimize gross phase separation with aging. Such compatibility can often be assessed, for example, by comparing melt flow characteristics of the styrene-diene block copolymer with that of the polypropylene, since similar viscosity properties aid in obtaining acceptable blending. The melt indices of both the polypropylene and the block copolymer can be measured by ASTM Test No. 1238 and the closer the melt indices, the more compatible the blend will be.

The compatibility of the block copolymer and the polypropylene will depend to a certain extent on molecular weight. If the molecular weights are comparable, compatibility will be improved.

Compatibility can also often be determined by using differential scanning calorimetry (DSC) to measure the melting points and glass transition temperatures of the polymer blend. If two glass transition temperatures are detected by DSC due to the constituent polymers in a blend, the blend is said to be incompatible. If a single glass transition temperature, intermediate between those of the component polymers, is detected, the blend is said to be compatible. Mechanically compatible blends represent a deviation from this generality, since they exhibit two glass transition temperatures but have finer morphology and are translucent. Such blends are useful in this invention.

The molecular weights of the polypropylene resin used can have a wide range, preferably in the range of 2 to 4 x $10^5$. Especially preferred resins have a melt index of from about 8 to 12 grams per 10 minutes. Such polypropylene resins are readily available from commercial sources such as Fina Oil and Chemical Company, Deer Park, TX; Exxon Chemical Americas, Houston, TX; Himont USA, Inc., Wilmington, DE; and Shell Chemical Co., Houston, TX.

Particular properties and sources of flexible styrene-diene block copolymers are disclosed in Kirk-Othmer, Encyclopedia of Chemical Technology, Vol. 8, pp. 626-640 (3rd Ed. John Riley & Sons, N.Y., N.Y.) which is incorporated herein by reference.

A desired balance of high tensile strengths and toughness can generally be obtained in the film base of tape of the invention when the polymers are mixed in a range of from about 1 to about 35 weight percent block copolymer. For especially high tensile strength and good toughness, the block copolymer is preferably present in an amount of 5 to 15 weight percent of the blend. For higher toughness and somewhat lesser tensile strength, the block copolymer is preferably present in an amount of 15 to 25 weight percent of the blend.

The film base for the tape of the invention is prepared from the above-described blends according to well-known extrusion casting techniques. The block copolymer and the crystalline isotactic polypropylene components are dry-blended in the desired ratio and fed into an extruder. The film typically can be made by extruding the molten polymer blend at a temperature of about 215°C to 275°C through a slot extrusion die and then into a nip between a rubber-covered roll and a water-cooled metal chill roll. In general, the chill roll temperature is 0°C to 80°C, depending upon the blend composition, roll contact time and desired tensile/modulus properties. Higher chill roll temperatures generally decrease the quench rate and thereby increase the crystallinity of the polypropylene which results in increased tensile strengths, but reduced toughness values and tear strengths. The chill roll may incorporate a shiny chrome finish, matte finish, or engraved pattern, including those disclosed in U.S. Patent No. 4,237,889. The chill roll contact time will generally be about 0.2 to 1.5 seconds, depending upon roll size, line speed, composition, and desired tensile/modulus properties. An increase in chill roll contact time generally results in reduced tensile strengths and increased toughness values and tear strengths.

The resulting film can be treated by any one or more known techniques (e.g. corona treatment, flame treatment and ultraviolet irradiation) to enhance surface bonding if desired and wound into a roll. The resulting film is untensilized, i.e., it has not been subjected to longitudinal or transverse stretching after extrusion, although molecular alignment may occur during processing and/or aging.

The incorporation of the block copolymer in no way precludes incorporation of pigments, fragrances, fillers, and/or dyes. Such materials may be incorporated in amounts up to a total of 10% of the weight of the film without detrimentally affecting the tape. In a preferred embodiment of this invention, pigment is incorporated to impart desired color and opacity to the tape.

The improved physical properties of the films such as tensile strength, tear strength and toughness are obtained in part by controlling the percent crystallinity and spherulite size of the polymer blends which are in turn controlled by the parameters defining the quench rate. The incorporation of flexible polymer results in a decrease in percent crystallinity and spherulite size in the polypropylene matrix. The crystallinity, and thus, tensile strength, modulus and stiffness can be recovered by decreasing the quench rate without sacrificing tensile impact toughness.

The crystallization behavior of isotactic polypropylene is well known. Very rapid rates of crystallization occur as the melt is initially quenched. Secondary crystallization, occurring at a much slower rate, is encountered during the first week after extrusion, after which the crystallization behavior remains relatively constant. The blending of block copolymer into polypropylene inhibits the crystallization of polypropylene to a degree dependent upon the nature and amount of added flexible polymer. However, the primary and secondary crystallization processes remain and must be accounted for.

The blended films useful e.g. as tape backings exhibit good tensile strength and tear strength with improved tensile impact toughness over that of known modified or unmodified homopolymer films or blended films. The films are useful as adhesive tape backings requiring high tensile strength and impact toughness, and have the following property characteristics:

1) a longitudinal yield tensile strength of at least 160 $kg/cm^2$ and preferably at least about 230 $kg/cm^2$, reaching up to 380 $kg/cm^2$ or more;

2) a tranverse yield tensile strength of at least 140 $kg/cm^2$ and preferably at least 210 $kg/cm^2$, reaching up to 360 $kg/cm^2$ or more;

3) a longitudinal tear strength of at least 50 g/ply, and preferably at least 150 g/ply, reaching up to 1000 g/ply or more;

4) a tensile impact toughness of greater than 20 mm/sec, preferably at least about 40 mm/sec and most preferably at least about 120 mm/sec, reaching up to 720 mm/sec or more.

In order to measure these critical property characteristics, the following test procedures were used. Films were aged and then tested a minimum of two weeks after extrusion to ensure equilibrium of properties.

Tensile Strength (ASTM D-882-81):

A 25.4 mm by 150 mm strip of film is mounted in a tensile testing machine e.g. an Instron Tensile Tester with the upper and lower jaws 25.4 mm apart. The jaws are then separated at the rate of 254 mm/minute until the yield point is reached. The machine direction (MD, longitudinal) and cross direction (CD, transverse) of the films were tested after equilibrium was reached (about 2 weeks). Yield tensile strength is reported in $kg/cm^2$.

Tear Strength:

One end of a specimen approximately 75 mm long and exactly 63 mm wide is positioned in a vertical plane with the long dimension extending horizontally, with the ends of the specimen gripped between a pair of fixed clamps horizontally spaced 2.5 mm from a pair of movable clamps which grip the other end of the test specimen. A 20 mm slit is made in the lower edge of the test specimen between the two pairs of clamps. A pendulum, carrying a circumferential graduated scale, is then allowed to fall freely, tearing the pre-cut test specimen along a continuation of the slit. A frictionally mounted pointer on the scale indicates the resistance in grams of the specimen to tearing. This test is commonly referred to as Elmendorf tear strength and values are reported in grams/ply.

Toughness:

Toughness is defined as the ability to withstand a high speed tensile force measured by the ductile/brittle transition. A 25.4 mm by 150 mm strip of film is mounted in a high speed tensile testing machine (MTS Systems) with the upper and lower jaws 25.4 mm apart. Samples are cut with a JDC

4

precision width cutter to reduce the number of edge flaws. A minimum of ten different strain rates are run for each film. Ten samples are run at each strain rate. Samples that show flaws by edge nicks, holes, or tearing rather than clean breaks are not counted among the ten. The number of brittle failures out of 10 good samples are recorded. Brittle failure is defined as samples that break before or at the yield point typically at elongations less than 30%. Ductile samples are defined as those exhibiting a yield point during the test and having elongations typically greater than 100%. The transition from ductile to brittle failure occurs over a range of strain rates. The "brittle onset" is defined as the strain rate at which two out of ten samples show brittle failure and the "brittle endpoint" is defined as the strain rate at which eight out of ten samples show brittle failure. Sample toughness is defined as the brittle onset strain rate or crosshead speed and is reported in mm/sec.

While any increase in toughness over 20 mm/sec is an improvement, toughness values of at least about 40 mm/sec represent a particular improvement. Furthermore, a film having toughness values of at least about 120 mm/sec yields a superior diaper closure tape.

The film backing is converted into tape by applying a coating of normally tacky and pressure-sensitive adhesive to one face, using conventional priming techniques if required. The coating can be a continuous layer or can be discontinuous such as a pattern coating. The adhesive may be transparent or, if desired, colored by incorporating dyes and pigments in a conventional manner. If the uncoated side of the film is exposed at the back of the tape, it may be desirable to apply a release coat such as a silicone polymer or any of several other conventional release coats thereover to facilitate unwinding rolls of the tape. A low release liner which is a silicone coated paper or film may also be used. The tape is normally made on wide sheets of film and then slit to desired widths and lengths and wound convolutely around cores. Diaper closure tapes are formed by cutting appropriately-sized strips (e.g., 2.5 cm x 7.5 cm) from the tape.

Where the tape is to be used in making closures for disposable diapers, it is important that the adhesive be capable of bonding firmly to the diaper cover, which is usually polyethylene film. Rubber-resin type pressure-sensitive adhesives are well-suited for this purpose, the "rubber" being either natural rubber or a synthetic copolymer. The type and amount of resin employed will depend on both the rubber and the degree of adhesiveness desired, appropriate adjustments being readily accomplished according to well-known procedures. Acrylate adhesives, e.g., a 94.5:5.5 isooctyl acrylate:acrylic acid copolymer as disclosed in U.S. Reissue Patent 24,906, may also be employed. Similarly, if the tape is to be converted into a closure for conventional cloth diapers, it may be desirable to utilize moisture-resistant adhesives of the type disclosed in U.S. Patent 4,074,004.

Disposable diapers normally have a liquid permeable layer designed to be placed next to the skin of the infant, a liquid impermeable layer which forms the outer portion of the diaper in use, and an absorbent layer between the liquid permeable layer and the liquid impermeable layer. The diaper is secured around the infant by a pressure-sensitive adhesive tape closure. A disposable diaper is generally rectangular in shape with closure tapes positioned near one longitudinal end thereof. Two tapes are attached near opposite corners of the outer surface of the liquid impermeable outer layer with a portion of each closure tape extending from the liquid impermeable layer. The portions which extend have a release liner in contact with the pressure-sensitive adhesive on each portion to protect the adhesive prior to fastening. In a preferred embodiment, a release liner is permanently adhered to the inner face of the disposable diaper and a portion of each closure tape is folded over the edges of the disposable diaper to contact the permanently adhered release liners.

This invention is further illustrated by the following examples denoted by numeral which should not be construed to unduly limit the invention. Comparative Examples are denoted by a letter. In the examples, all parts and percentages are by weight unless otherwise indicated.

Examples 1 and 2

Film samples were prepared using crystalline isotactic polypropylene homopolymer (PP) ("Dypro" 8771, 95% isotactic polypropylene, density 0.905, available from Fina Oil and Chemical Company, Deer Park, TX) and a styrene/isoprene/styrene copolymer available from the Shell Chemical Co.

The polymers were dry-blended and heated to the molten state. The molten polymer blend was extruded through a slot extrusion die and then into the nip between a silicone rubber-covered roll having an average roughness (Ra) of 20 and an average maximum profile height (Rpm) of 115, and a water cooled metal chill roll having an Ra of 250 and an Rpm of 220, to effect quenching and provide a matte finish. The roughness properties, Ra and Rpm, were measured with a Surtronic 3, available from Taylor-Hobson, Leicester, England. The temperature of the chill roll and the chill roll contact time are shown in Table 1. the surfaces of the resulting film were primed using conventional corona discharge techniques immediately after extrusion and then aged at room temperature (70°F, 21°C) for at least two weeks to allow the physical properties to reach equilibrium prior to testing for physical characteristics. The compositions, quench conditions, and film properties are set forth in Table 1 below.

## TABLE 1

| Run No. | 1 | 2 |
|---|---|---|
| % PP | 90 | 95 |
| % Flexible Polymer | 10(SIS) | 5(SIS) |
| Melt Temp. (°C) | 232 | 221 |
| Chill Roll Temp. (°C) | 18 | 57 |
| Chill Roll Contact time (sec) | 0.37 | 0.73 |
| Caliper (microns) | 105 | 76 |
| Tensile Strength MD (kg/cm$^2$) | 254 | 295 |
| Tensile Strength CD (kg/cm$^2$) | 232 | 270 |
| Tear Strength MD (gm/ply) | 174 | 56 |
| Toughness CD (mm/sec) | 300 | 106 |

## Claims

1. A pressure-sensitive adhesive tape comprising a backing of a substantially untensilized, tough, ductile film, and a coating of a pressure-sensitive adhesive on one side of said film; said film comprising a blend of crystalline isotactic polypropylene and a compatible styrene-diene block copolymer having a flexural modulus lower than that of said isotactic polypropylene, said film having
   1) a longitudinal yield tensile strength of at least about 160 kg/cm$^2$;
   2) a transverse yield tensile strength of at least about 140 kg/cm$^2$;
   3) a longitudinal tear strength of at least about 50 g/ply; and
   4) a tensile impact toughness of greater than about 20 mm/sec;

2. A tape according to claim 1 wherein said styrene-diene block copolymer has a flexural modulus lower than 500 MPa.

3. A tape according to claim 2 wherein said styrene-diene block copolymer has a flexural modulus of from 40 MPa to 400 MPa.

4. A tape according to any of claims 1-3 wherein said tensile impact toughness is at least 120 mm/sec.

5. A tape of any of claims 1-4 wherein said film is comprised of 65 to 99 weight percent crystalline isotactic polypropylene and 35 to 1 weight percent of said styrene-diene block copolymer.

6. A tape according to any of claims 1-4 wherein said film is comprised of 75 to 85 weight percent crystalline isotactic polypropylene and 15 to 25 weight percent of said styrene-diene block copolymer.

7. A tape according to any of claims 1-4 wherein said film contains 5 to 15 weight percent of said styrene-diene block copolymer and 95 to 85 weight percent crystalline isotactic polypropylene.

8. A tape according to any preceding claim wherein said styrene-diene block copolymer is a styrene/isoprene styrene block copolymer.

9. A tape according to any preceding claim wherein said longitudinal tear strength is at least about 150 g/ply.

10. A disposable diaper to which tape as claimed in any of claims 1-9 is attached as a diaper closure means.

**Patentansprüche**

1. Haftklebeband, umfassend einen Träger einer im wesentlichen nichtgestreckten, zähen, dehnbaren Folie und eine Beschichtung eines Haftklebers auf einer Seite der Folie, wobei die Folie eine Mischung von kristallinem, isotaktischen Polypropylen und einem kompatiblen Styrol-Dien-Blockcopolymer mit einem niedrigeren Biegemodul als dem des isotaktischen Polypropylens umfaßt und die Folie aufweist:
(1) eine Zugfestigkeit in Längsrichtung von mindestens 160 kg/cm$^2$;
(2) eine Zugfestigkeit in Querrichtung von mindestens 140 kg/cm$^2$;
(3) eine zerreißfestigkeit in Längsrichtung von mindestens 50 g/Lage und
(4) eine Zugschlagzähigkeit größer als 20 mm/s.

2. Band nach Anspruch 1, bei welchem das Styrol-Dien-Blockcopolymer einen Biegemodul von weniger als 500 MPa hat.

3. Band nach Anspruch 2, bei welchem das Styrol-Dien-Blockcopolymer einen Biegemodul von 40 MPa bis 400 MPa hat.

4. Band nach einem der Ansprüche 1 bis 3, bei welchem die Zugschlagzähigkeit mindestens 120 mm/s beträgt.

5. Band nach einem der Ansprüche 1 bis 4, bei welchem die Folie 65 bis 99 Gewichtsprozent kristallines isotaktisches Polypropylen und 35 bis 1 Gewichtsprozent des Styrol-Dien-Blockcopolymers aufweist.

6. Band nach einem der Ansprüche 1 bis 4, bei welchem die Folie 75 bis 85 Gewichtsprozent kristallines isotaktisches Polypropylen und 15 bis 25 Gewichtsprozent des Styrol-Dien-Blockcopolymers aufweist.

7. Band nach einem der Ansprüche 1 bis 4, bei welchem die Folie 5 bis 15 Gewichtsprozent des Styrol-Dien-Blockcopolymers und 95 bis 85 Gewichtsprozent kristallines isotaktisches Polypropylen aufweist.

8. Band nach einem der vorstehenden Ansprüche, bei welchem das Styrol-Dien-Blockcopolymer ein Styrol/Isopren-Styrol-Blockcopolymer ist.

9. Band nach einem der vorstehenden Ansprüche, bei welchem die Zerreißfestigkeit in Längsrichtung mindestens 150 g/Lage beträgt.

10. Wegwerfwindel mit daran als ein Windelverschluß angebrachtem Band nach einem der Ansprüche 1 bis 9.

**Revendications**

1. Ruban adhésif sensible à la pression, comprenant un support formé d'un film essentiellement non étiré, solide et ductile, et un revêtement d'adhésif sensible à la pression sur une face dudit film, ledit film comprenant un mélange de polypropylène cristallin isotactique et un copolymère séquencé styrène-diène compatible, ayant un module de flexion inférieur à celui dudit propylène isotactique, ledit film ayant :

    1) une limite de résistance à la rupture par traction longitudinale d'au moins environ 160 kg/cm$^2$ ;

    2) une limite de résistance à la rupture par traction transversale d'au moins environ 140 kg/cm$^2$ ;

    3) une résistance au déchirement longitudinal d'au moins environ 50 g/pli ; et

    4) une solidité au choc en traction supérieure à environ 20 mm/s.

2. Ruban selon la revendication 1, dans lequel ledit copolymère séquencé styrène-diène a un module de flexion inférieur à 500 MPa.

3. Ruban selon la revendication 2, dans lequel ledit copolymère séquencé styrène-diène a un module de flexion de 40 MPa à 400 MPa.

4. Ruban selon l'une quelconque des revendications 1-3, dans lequel ladite solidité au choc en traction est d'au moins 120 mm/s.

5. Ruban selon l'une quelconque des revendications 1-4, dans lequel ledit film est formé de 65 à 99 % en poids de polypropylène cristallin isotactique et 35 à 1 % en poids dudit copolymère séquencé styrène-diène.

6. Ruban selon l'une quelconque des revendications 1-4, dans lequel ledit film est formé de 75 à 85 % en poids de polypropylène cristallin isotactique et 15 à 25 % en poids dudit copolymère séquencé styrène-diène.

7. Ruban selon l'une quelconque des revendications 1-4, dans lequel ledit film contient 5 à 15 % en poids dudit copolymère séquencé styrène-diène et 95 à 85 % en poids de polypropylène cristallin isotactique.

8. Ruban selon l'une quelconque des revendications précédentes, dans lequel ledit copolymère séquencé styrène-diène est un copolymère séquencé styrène/styrène isoprène.

9. Ruban selon l'une quelconque des revendications précédentes, dans lequel la résistance au déchirement longitudinal est au moins d'environ 150 g/pli.

10. Couche jetable comprenant un ruban tel que revendiqué dans l'une quelconque des revendications 1 à 9 et constituant des moyens de fermeture pour ladite couche.